# EUROPEAN PATENT APPLICATION

(11) **EP 3 424 490 A1**
(43) Date of publication of application: **09.01.2019**
(21) Application number: 17305881.9
(22) Date of filing: 06.07.2017
(51) Int. Cl.: A61K 9/00, A61M 16/12, A61K 33/00

(54) **GAS MIXTURES CONTAINING XENON AND ARGON AT LOW CONCENTRATION AS ORGAN PROTECTANT, IN PARTICULAR NEUROPROTECTANT**

(71) Applicant: Monatomics Technology, 75016 Paris (FR)
(72) Inventor: DAVID, Hélène, Québec G0A 3Z0 (CA)
(74) Representative: Ex Materia

(57) **Abstract**

The present invention relates to low concentration synergistic Xenon-Argon gas mixtures as organ protectant, in particular as neuroprotectant. Specifically, the present invention relates to a pharmaceutical composition comprising a gas mixture of xenon and argon for use as organ protectant, in particular as neuroprotectant, wherein
- the volume proportion of xenon is between 5 and 25%,
- the volume proportion of argon is between 5 and 25%, and
- the pharmaceutical composition further comprises a gas complement to reach 100% volume proportion.

More specifically, the pharmaceutical composition of the invention is useful for treating and/or preventing and/or slowing the degradation of the structure and/or function of organ cells (in particular neurons), in mammals suffering a decompression accident, neonatal hypoxia-ischemia, stroke, traumatic brain injury, cardiac arrest, renal dysfunction, neurocognitive disorders, post-traumatic stress disorder (PTSD), neurodegenerative diseases, and psychiatric disorders.

## Description

### Technical Field

The present invention relates to the field of organ protection, particularly neuroprotection, and gas mixtures for use as organ-protectant, in particular as neuroprotectant.

### Technological Background

Organ protection refers to the relative preservation of the cellular structure and function of an organ which has become dysfunctional due to a primary or secondary, direct or indirect, insult. Neuroprotection is a particular case of organ protection, referring to the relative preservation of the structure and/or function of nerve cells, *i.e.* neurons. It is a widely-explored treatment option for many central nervous system (CNS) disorders including stroke, traumatic brain injury, spinal cord injury, neurodegenerative diseases, the acute and long-term effects of drug of abuse consumption, and many psychiatric diseases. Despite differences in symptoms, these CNS disorders share more or less common mechanisms such as increased levels of oxidative stress, protein aggregation, mitochondrial dysfunction, inflammatory cascade, decreased γ-amino-butyric acid (GABA)-mediated neurotransmission activity, and increased N-methyl-D-aspartate (NMDA) glutamate-mediated neurotransmission.
Organ protection aims at slowing or preventing disease progression by stopping or slowing cell death. Neuroprotection aims at slowing or preventing disease progression by stopping or slowing neuronal death.
Inert gases, such as nitrous oxide, xenon, argon, helium, neon, and mixtures thereof, are now well-known to show neuroprotective action in animal models of hypoxic-ischemic brain insults, brain trauma, and drug abuse. Particularly, studies in models of hypoxic-ischemic brain insults have clearly demonstrated the neuroprotective potential and lack of adverse toxic effects of the chemically and metabolically inert gases xenon and argon [1-7].
Among the inert gases and other molecules, xenon is considered the gold standard due to its potent preclinical effects at providing organ protection or neuroprotection and its lack of adverse side effect. However, the scarcity and excessive cost of production of xenon limit its widespread clinical development. In contrast, argon is a cost-efficient gas but, unfortunately, it is far less potent than xenon at providing organ protection, particularly neuroprotection [3,6]. Patent US 8,435,569 relates to the use of at least one thrombolytic agent (A), such as the human recombinant form of tissue-type plasminogen activator (rt-PA), and at least one gas (B) selected from the group consisting of nitrous oxide, argon, xenon, helium, neon, and mixtures thereof, for the preparation of a combined pharmaceutical composition for treating ischemia. However, Patent US 8,435,569 does not disclose any synergistic effects in terms of neuroprotection between the above-mentioned gases, particularly between argon and xenon. To date, there thus remains a need for a cost-efficient gas mixture useful in organ protection, and more specifically as neuroprotectant.

### Summary of the invention

Surprisingly, the Inventors discovered that gas mixtures containing low concentrations of xenon and argon provide organ protection. More specifically, these synergistic mixtures provide neuroprotection at the same level as xenon alone at the optimal concentration of 50 vol%.
Without wishing to be bound by theory, it is hypothesized that the synergistic neuroprotective effects of xenon and argon at low concentrations arise from their complementary mode of action: while xenon is thought to provide neuroprotection mainly through antagonism at the glutamatergic N-methyl-D-aspartate receptor (NMDA) [8], which is the main excitatory receptor in the brain, argon is believed to act mainly by potentiating the γ-aminobutyric acid type A (GABA-A) [9] receptor that is the main inhibitory receptor in the brain.
The inventors indeed investigated, using *ex vivo* and *in vivo* preparations, the effects of gas mixtures containing equimolar concentrations of xenon (Xe) and argon (Ar) of 5 vol% (Xe-Ar-5), 15 vol% (Xe-Ar-15), 25 vol% (Xe-Ar-25) and/or 37.5 vol% (Xe-Ar-37.5) on cell injury induced by oxygen and glucose deprivation (OGD) in brain slices and brain damage in rats subjected to an intracerebral injection of NMDA. Particularly, the inventors found that Xe-Ar-15 > Xe-Ar-25 > Xe-Ar-37.5 at providing neuroprotection both in brain slices and rats that were given NMDA. Interestingly, in both cases, Xe-Ar-15 provides neuroprotection as potent as xenon alone at 50 vol%, generally thought to be the optimal neuroprotective concentration of xenon. However, it was found that administration of an equimolar concentration of xenon and argon at 5 vol% in rats subjected to an intracerebral NMDA injection fails to provide neuroprotection.

Therefore, in a first aspect, the present invention relates to a pharmaceutical composition comprising a gas mixture of xenon and argon for use as an organ-protective, in particular a neuroprotective, agent wherein
- the volume proportion of xenon is between 5 and 25%,
- the volume proportion of argon is between 5 and 25%, and
- the pharmaceutical composition further comprises a gas complement to reach 100% volume proportion.
In a second aspect, the present invention relates to the use of a pharmaceutical composition comprising a gas mixture of xenon and argon, wherein
- the volume proportion of xenon is between 5 and 25%,
- the volume proportion of argon is between 5 and 25%, and
- the pharmaceutical composition further comprises a gas complement to reach 100% volume proportion,
to manufacture a medicament for providing organ protection, in particular neuroprotection.
In a third aspect, the present invention relates to a method for providing organ protection, in particular neuroprotection, comprising administering to a patient in need thereof an effective dose of a pharmaceutical composition comprising a gas mixture of xenon and argon, wherein
- the volume proportion of xenon is between 5 and 25%,
- the volume proportion of argon is between 5 and 25%, and
- the pharmaceutical composition further comprises a gas complement to reach 100% volume proportion.
In the present invention, "organ protection" is understood as the relative preservation of the cellular structure and function of an organ which has become dysfunctional due to a primary or secondary, direct or indirect, insult. Providing organ protection thus aims at treating and/or slowing and/or preventing disease progression by stopping or at least slowing organ cell death in the.
In the present invention, "neuroprotection" is a specific type of organ protection, understood as the relative preservation of the neuronal structure and/or function. Providing neuroprotection thus aims at treating and/or slowing and/or preventing disease progression and secondary injuries by stopping or at least slowing neuronal death (or loss of neurons).
In the present invention, the "patient" is an animal such as a mammal, and preferably a human. The present invention has thus applications in both human and veterinary medicine.
In the following, the term "use as an organ protectant" is considered equivalent to a "method for providing organ protection", and are both meant to refer to a therapeutic protocol aimed at providing organ protection to the patient.
Also, in the following, the term "use as an neuroprotectant" is considered equivalent to a "method for providing neuroprotection", and are both meant to refer to a therapeutic protocol aimed at providing neuroprotection to the patient.
As used herein, the "volume proportion" of a gas is a percentage calculated on the basis of the volume of the gaseous composition as a whole. Where appropriate, the volume proportion is based on the volume of the decompressed, reconstituted, gaseous composition as a whole. In the following, the "concentration of a gas" is understood as the volume proportion of said gas.

### Detailed Description

The present invention concerns a pharmaceutical composition comprising a gas mixture of xenon and argon for use as an organ-protectant, in particular as a neuroprotectant, wherein
- the volume proportion of xenon is between 5 and 25%, in particular between 8 and 22%, such as between 10% and 20%, for instance it is of 10%, 15%, 20%, or 25%,
- the volume proportion of argon is between 5 and 25%, in particular between 8 and 22%, such as between 10% and 20%, for instance it is of 10%, 15%, 20%, or 25%, and
- the pharmaceutical composition further comprises a gas complement to reach 100% volume proportion.
Advantageously, the xenon/argon volume ratio is between 4/1 and 1/4, preferably between 3/1 and 1/3, more preferably between 2/1 and 1/2, most preferably it is of 1/1 (*i.e.* equimolar mixtures).
In a particular embodiment, the pharmaceutical composition comprises a gas mixture of xenon and argon wherein:
- the volume proportion of xenon is between 10 and 20%, and
- the volume proportion of argon is between 10 and 20%. In this embodiment, the gas mixture is advantageously equimolar.
In a particular example, the volume proportion of xenon is 15%, and the volume proportion of argon is 15%.

The pharmaceutical composition of the invention is intended for any appropriate route of administration, preferably through inhalation. The synergistic composition of the invention is thus preferably inhalable. It thus comprises a gas complement. Typically, the gas complement comprises oxygen, nitrogen, helium, hydrogen, neon or a mixture thereof. Of course, only stable, non-explosive gas mixtures are contemplated. In particular, in the present invention, when the gas complement comprises hydrogen and oxygen, these gases are in stable, non-explosive proportions.
Preferably, the gas complement comprises oxygen, typically in a volume proportion of between 20% and 50% (such as between 25% and 45% or between 30% and 40%, for instance the volume proportion of oxygen is 20%, 25%, 30%, 35%, 40%, 45% or 50%), and the remainder of the gas in the gas complement is advantageously nitrogen, helium, hydrogen, neon or a mixture thereof, preferably nitrogen, helium, neon or a mixture thereof, most preferably helium. Most preferably, the gas complement comprises oxygen typically in a volume proportion of between 20% and 50%, and the remainder of the gas in the gas complement is helium.
In a first embodiment, the pharmaceutical composition is preconditioned as a compressed gas mixture, and administered to the patient as a decompressed gas mixture.
In a second embodiment, the pharmaceutical composition administered to the patient is obtained by mixing argon, xenon and the gas complement in a gas mixer (and administered to the patient as a mixture of decompressed gas). In this second embodiment, argon, xenon and the gas complement are preconditioned as compressed gas in individual pressurized compartments, and decompressed prior to or upon mixing. Of note, the gas complement may itself be a gas mixture (typically comprising oxygen), which may also be obtained by mixing different gases into the gas mixer. Therefore, in this second embodiment, argon, xenon, oxygen and optionally other individualized gas tanks are connected to the gas mixer, wherein they are mixed in the desired volume proportions upon or after decompression. Preferably, each gas is decompressed prior to being introduced into the gas mixer.

As explained above, organ protection refers to the relative preservation of the cellular structure and function of an organ that has became dysfunctional due to a primary or secondary, direct or indirect, insult. In particular, neuroprotection refers to the relative preservation of the neuronal structure and/or function.
Therefore, the method for providing organ protection, in particular neuroprotection, aims at treating and/or preventing and/or slowing the degradation of the structure and/or function of the cells of an organ. In the specific case of neuroprotection, the organ's cells are neurons. The degradation of the structure and/or function of the cells of an organ (in particular neurons) may be caused by a disease or condition selected from the group consisting of: cardiac arrest, stroke, neonatal hypoxia-ischemia, renal dysfunction, traumatic brain injury, post-traumatic stress disorder (PTSD), neurocognitive disorders (such as those being the consequence of cardiac arrest), neurodegenerative diseases (such as Alzheimer's Disease or Parkinson's Disease), and psychiatric disorders (such as schizophrenia, depression, bipolar disorder, addiction, anxiety disorders, attention deficit disorder and/or hyperactivity disorder). Specifically, the degradation of the neuronal structure and/or function of a nervous organ (for example the brain) may be caused by: stroke, neonatal hypoxia-ischemia, traumatic brain injury, neurocognitive disorders (such as those being the consequence of cardiac arrest), neurodegenerative diseases (such as Alzheimer's Disease or Parkinson's Disease), and psychiatric disorders such as schizophrenia, depression, bipolar disorder, addiction, anxiety disorders, attention deficit disorder and/or hyperactivity disorder.
Alternatively, the degradation of the cellular (in particular neuronal) structure and/or function of an organ may be caused by a decompression accident (also known as decompression sickness or decompression illness).
The "effective dose" or "efficient dose" of a composition of the invention, comprising a synergistic mixture of xenon and argon, varies as a function of numerous parameters such as, for example, the route of administration and the weight, the age, the sex, the advancement of the pathology to be treated and the sensitivity of the subject or patient to be treated.
In an advantageous embodiment of the invention, the method for providing organ protection (in particular neuroprotection) does not comprise administering a pharmaceutical agent with thrombolytic properties (even if initially developed for other medical use, such as N-acetylcysteine or N-Acetyl-L-cysteine), such as a thrombolytic agent, for example streptokinase, urokinase, reteplase, tenecteplase, or alteplase (human recombinant tissue-type plasminogen activator or rt-PA). In particular, the method does not comprise giving rt-PA to the patient, before, together with or after administering the synergistic pharmaceutical composition of the invention.

### Description of the figures

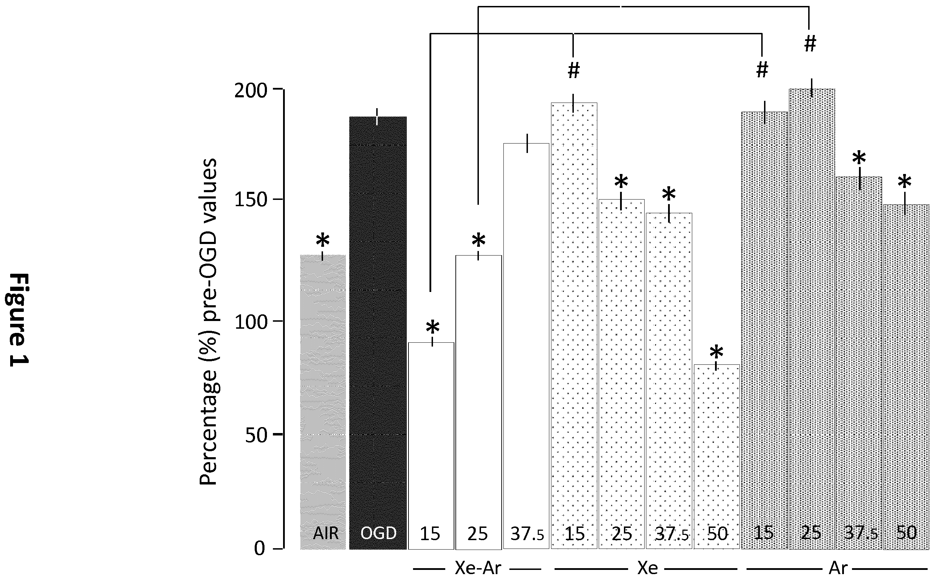

Figure 1. Effects of gas mixtures containing equimolar concentrations of xenon and argon of 15 vol% to 37.5 vol% on the release of lactate dehydrogenase (LDH) induced by oxygen-glucose deprivation (OGD) expressed as a percentage of pre-OGD values. Xe-Ar-15: gas mixture with equimolar concentration of xenon and argon of 15 vol%; Xe-Ar-25: gas mixture with equimolar concentration of xenon and argon of 25 vol%; Xe-Ar-37.5: gas mixture with equimolar concentration of xenon and argon of 37.5 vol%. Xe-Ar-15 approximately reduced OGD-induced LDH release in a manner similar to that of xenon at 50 vol%. Significant differences are marked by stars and sharp. * *P* < 0.0001 *vs* OGD slices; ^{#} P < 0.0001 vs xenon or argon alone at 15 vol% or 25 vol%.

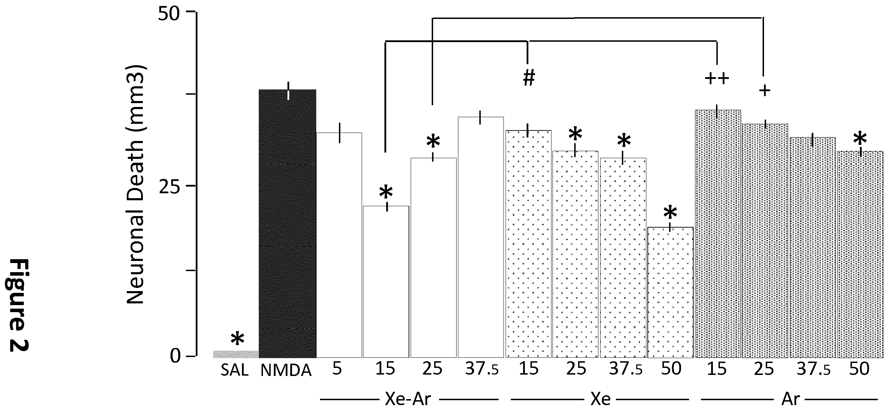

Figure 2. Effects of gas mixtures containing equimolar concentrations of xenon and argon of 15 vol% to 37.5 vol% on brain damage induced by intracerebral injection of N-methyl-D-aspartate (NMDA) expressed in mm³. Xe-Ar-5: gas mixture with equimolar concentration of xenon and argon of 5 vol%; Xe-Ar-15: gas mixture with equimolar concentration of xenon and argon of 15 vol%; Xe-Ar-25: gas mixture with equimolar concentration of xenon and argon of 25 vol%; Xe-Ar-37.5: gas mixture with equimolar concentration of xenon and argon of 37.5 vol%. Xe-Ar-15 approximately reduced NMDA-induced brain damage in a manner similar to that of xenon at 50 vol%. Significant differences are marked by stars, sharps, or plus symbols. * *P <* 0.0001 *vs* OGD slices; ^{#} *P* < 0.002 vs xenon at 15 vol%; ⁺⁺ *P* < 0.02 vs argon at 15 vol%; ⁺ *P* < 0.05 vs argon at 25 vol%.

### EXAMPLES

The present invention is illustrated by the following examples, which are not to be construed as limiting the invention in any way.

### MATERIALS AND METHODS

### Animals

All animal-use procedures were performed in accordance with the Declaration of Helsinki and were within the framework of the French legislation for the use of animals in biomedical experimentation. Adult male Sprague-Dawley rats (Janvier, Le Genest Saint-Isle, France) weighing 250-280 g were used. Before being used, rats were housed at 21 ± 0.5°C in Perspex home cages with free access to food and water. Light was maintained on a light/dark reverse cycle, with lights on from 8:00 PM to 8:00 AM.

### Preparation and incubation of brain slices

Rats were killed by decapitation under halothane anesthesia. The brains were removed and placed in ice-cold freshly prepared artificial cerebrospinal fluid (aCSF). Coronal brain slices (400 µm thickness) including the striatum (anteriority: from +1.2 to +2 mm from bregma) were cut using a tissue chopper (Mickie Laboratory Engineering Co., Gomshall, Surrey, UK).

### Measurement of cell injury with lactate dehydrogenase activity assay

The effects of gas mixtures containing xenon and argon on the release of lactate dehydrogenase (LDH) induced by OGD were assessed as described previously [6]. Before being used, brain slices were transferred into individual vials with 1.3 ml of freshly prepared oxygenated aCSF containing 120 mM NaCl, 2 mM KCl, 2 mM CaCl₂, 26 mM NaHCO₃, 1.19 mM MgSO₄, 1.18 mM KH₂PO₄, 11 mM d-glucose, and 30 mM HEPES and allowed to recover at room temperature for 45 min. Then, brain slices were placed at 36 ± 0.5°C into individual vials containing 1.3 ml of freshly prepared aCSF, saturated, and continuously bubbled with 100 % oxygen (25 ml/min per vial). After a 30-min period, the incubation aCSF solution was renewed with oxygenated aCSF maintained at 36°C, and the slices were then incubated for 1 h to allow recording of basal levels of LDH. Whereas control slices were incubated for an additional 20-min period in the same conditions, those corresponding to the ischemic group were incubated in a glucose-free solution, saturated, and continuously bubbled with 100 % nitrogen (OGD slices). After this 20-min period of OGD, to mimic reperfusion and treatment, the medium was replaced in all groups with freshly prepared aCSF solution, saturated and continuously bubbled with either medical air or gas mixtures containing xenon and argon in volume proportions as described below (see gas pharmacology section; n = 20-36).

### NMDA-induced neuronal death in vivo

The effects of gas mixtures containing xenon and argon on the catalytic activity of tPA were assessed as described previously [6]. On the day of surgery, rats were anesthetized with 1.5% halothane in oxygen alone in an anesthesia box and mounted on a stereotactic apparatus with the incisor bar set at 3.9 mm below the horizontal zero. A burr hole was drilled and a micropipette (less than 10 µm at the tip) was lowered into the right striatum (anterior, 0.6 mm; lateral, 3.0 mm; ventral, 5.8 mm, from bregma) to allow injection of 70 nmol of NMDA in 1 µl of phospahte buffered solution (pH 7.4) over a 2-min period. After an additional 5-min period, the micropipette was removed, and the wounds were then sutured. During surgery, body temperature was kept at 37 ± 0.5°C with a feedback-controlled thermostatic heating pad (Harvard Apparatus Limited, Edenbridge, UK). The animals woke up in their home cage after about 10 min, where they were given free access to food and water. Sixty minutes after administration of NMDA, rats were treated with medical air or gas mixtures containing xenon and argon in volume proportions as described below (see gas pharmacological section). The number of rats per group was n = 7-11.

### Gas Pharmacology

Xenon, argon, nitrogen, and oxygen were purchased from Air Liquide Santé (Paris, France). Medical air composed of 75% nitrogen and 25% oxygen, and xenon and argon mixtures containing 37.5% xenon and 37.5% argon (Xe-Ar-37.5), 25% xenon and 25% argon (Xe-Ar-25), 15% xenon and 15% argon (Xe-Ar-15), and/or 5% xenon and 5% argon (Xe-Ar-5) - with 25% oxygen and the remainder being nitrogen when needed - were obtained using computer-driven gas mass flowmeters (Aalborg) of 1% accuracy, and an oxygen analyzer to double check that the gas mixtures used were not hypoxic.

### Statistical Analysis

Data were analyzed. Data are given as the mean ± the standard error to the mean. The effects of gas mixtures containing xenon and argon were analyzed with Statview software (SAS Institute, Cary, NC) and compared to those of control experiments, xenon, and argon using non-parametric Mann-Whitney U-test.

### RESULTS

First, the neuroprotective effects of gas mixtures containing xenon and argon at equimolar concentrations of 15% to 37.5% was investigated in brain slices exposed to OGD, a model of brain ischemia. OGD induced an increase in LDH release compared to control slices (*P* < 0.0001). As illustrated in Fig. 1, it was found that Xe-Ar-15 > Xe-Ar-25 > Xe-Ar-37.5 at reducing the release of LDH, a marker of cell injury, in brain slices exposed to OGD. Xe-Ar-15 decreased LDH (*P* < 0.0001 *vs* Air-treated OGD slices) in a manner similar to that of xenon at 50%. This led to a significant difference between Xe-Ar-15 and xenon at 15% (*P* < 0.0001), which had no effect by itself on OGD-induced LDH release, and Xe-Ar-15 and argon at 15% (*P* < 0.0001), which also had no effect by itself on OGD-induced LDH release. Xe-Ar-25 decreased OGD-induced LDH release (*P* < 0.0001) with similar amplitude than xenon alone at 25-37.5%. This led to a significant difference between Xe-Ar-25 and argon at 25% (P < 0.0001), which had no effect by itself on OGD-induced LDH release, but not with xenon at 25%, thereby indicating that the neuroprotective effect of Xe-Ar-25 mainly resulted from the presence of xenon in the gas mixture. In contrast with Xe-Ar-15 and Xe-Ar-25, we found that Xe-Ar-37.5 failed to decrease LDH release in OGD slices.

Second, the neuroprotective effects of gas mixtures containing xenon and argon at equimolar concentrations of 5% to 37.5% was investigated in rats that were given intracerebral injection of NMDA. Intracerebral injection of NMDA in rats treated with air induced brain damage compared to air-treated controls injected with saline (*P <* 0.0001). As shown in Fig. 2, it was found Xe-Ar-15 > Xe-Ar-25 > Xe-Ar-5 > Xe-Ar-37.5 at reducing brain damage in rats injected with NMDA. Xe-Ar-15 reduced NMDA-induced brain damage (*P <* 0.001) in a manner similar to that of xenon alone at 50%. This led to a significant difference between Xe-Ar-15 and xenon at 15% (*P* < 0.002), which had no effect by itself on NMDA-induced brain damage, and Xe-Ar-15 and argon at 15% (P < 0.02), which also had no effect by itself on NMDA-induced brain damage. Likewise, Xe-Ar-25 reduced NMDA-induced brain damage (P < 0.02) with similar amplitude than xenon alone at 25-37.5%. This led to a significant difference between Xe-Ar-25 and argon at 25% (P < 0.05), which had no effect by itself on NMDA-induced brain damage, but not with xenon at 25%, thereby indicating that the neuroprotective effect of Xe-Ar-25 mainly resulted from the presence of xenon in the gas mixture. In contrast with Xe-Ar-15 and Xe-Ar-25, we found that Xe-Ar-5 and Xe-Ar-37.5 failed to show neuroprotection in rats injected with intracerebral NMDA.

### DISCUSSION

In the present study, it was shown that gas mixtures containing low concentrations of xenon and argon of 15 vol% (Xe-Ar-15) or 25 vol% (Xe-Ar-25) of each gas can provide neuroprotection. Particularly, it was found that gas mixtures containing Xe-Ar-15 reduced OGD-induced cell injury and NMDA-induced brain damage in a similar extent than xenon alone at 50 vol%, thereby indicating that Xe-Ar-15 has potent neuroprotective effects. These potent neuroprotective effects of Xe-Ar-15, together with the fact that neither xenon alone at 15 vol% nor argon alone at 15 vol% (data not shown) had neuroprotective properties, is believed to be due to a synergism between inhibition by xenon of the NMDA receptor [8], which is the main excitatory receptor in the brain, and activation by argon of the GABA-A receptor [9], which is the main inhibitory receptor in the brain. Because Xe-Ar-15 is as potent as xenon alone at 50 vol%, and much more potent than argon alone at any of the concentrations used, at providing neuroprotection, it is likely that the mechanisms by which Xe-Ar-15 acts is mainly mediated through the NMDA receptor, which is the main neuronal target of xenon [9]. In contrast with the potent neuroprotective effects of Xe-Ar-15, it was found that Xe-Ar-5 and Xe-Ar-37.5 failed to provide neuroprotection. The reason for the lack of effect of Xe-Ar-5 is with no doubt the too small concentration of gas used. So far Xe-Ar-37.5 is concerned the more plausible cause for this lack of effect of Xe-Ar-37.5 is a too potent general reduction in neuronal activity, which could be equivalent to narcosis or anesthesia in vivo. Support for this is the fact that at concentrations above Xe-Ar-15 neuroprotection decreased as a function of the concentration of Xe-Ar used (Xe-Ar-15 > Xe-Ar-25 > Xe-Ar-37.5). Further support for this are previous data that have shown that xenon provided neuroprotection at subanesthetic concentrations of 37.5 vol% or 50 vol% but not at high anesthetic concentrations of or above 75 vol% [5,6].

### REFERENCES

1. Ryang YM, Fahlenkamp AV, Rossaint R, Loetscher PD, Beyer C, et al. Neuroprotective effect of argon in an in vivo model of transient middle cerebral artery occlusion in rats. Crit Care Med 2011; 39:1448-1453.
2. Jawad N, Rizvi M, Gu J, Adeyi O, Tao G, Maze M, Ma D. Neuroprotection (and lack of neuroprotection) afforded by a series of noble gases in an in vitro model of neuronal injury. Neurosci Lett 2009; 460:232-236.
3. David HN, Haelewyn B, Degoulet M, Colomb DG Jr, Risso JJ, Abraini JH. Ex vivo and in vivo neuroprotection induced by argon when given after an excitotoxic or ischemic insult. PLoS One 2012; 7:e30934.
4. Homi HM, Yokoo D, Ma D, Warner DS, Franks NP, Maze M, Grocott HP. The neuroprotective effect of xenon administration during transient middle cerebral artery occlusion in mice. Anesthesiology 2003; 99: 876-881.
5. David HN, Léveillé F, Chalzaviel L, MacKenzie ET, Buisson A, Lemaire M, Abraini JH. Reduction of ischemic brain damage by nitrous oxide and xenon. J Cereb Blood Flow Metab 2003; 23:1168-1173.
6. David HN, Haelewyn B, Rouillon C, Lecocq M, Chazalviel L, Apiou G, Risso JJ, Lemaire M, Abraini JH. Neuroprotective effects of xenon: a therapeutic window of opportunity in rats subjected to transient cerebral ischemia. FASEB J 2008; 22:1275-1286.
7. Dingley J, Tooley J, Porter H, Thoresen M. Xenon provides short-term neuroprotection in neonatal rats when administered after hypoxia-ischemia. Stroke 2006; 37, 501-506.
8. Abraini JH, Kriem B, Balon N, Rostain JC, Risso JJ. GABA neuropharmacological investigations of narcosis produced by nitrogen, argon, and nitrous oxide. Anesth Analg 2003, 96:746-749.
9. Franks NP, Dickinson R, de Sousa SLM, Hall AC, Lieb WR. How does xenon produce anesthesia? Nature 1998; 396:324

## Claims

1. A pharmaceutical composition comprising a gas mixture of xenon and argon for use in a method for providing organ-protection, wherein
- the volume proportion of xenon is between 5 and 25%,
- the volume proportion of argon is between 5 and 25%, and
- the pharmaceutical composition further comprises a gas complement to reach 100% volume proportion.

2. The pharmaceutical composition for use of claim 1, wherein the method is for providing neuroprotection.

3. The pharmaceutical composition for use of claim 1 or 2, wherein the method does not comprise administering a pharmaceutical agent with thrombolytic properties, such as a thrombolytic agent in particular, streptokinase, urokinase, alteplase, reteplase or tenecteplase.

4. The pharmaceutical composition for use of any of claims 1 to 3, wherein
- the volume proportion of xenon is between 10 and 20%, and
- the volume proportion of argon is between 10 and 20%.

5. The pharmaceutical composition for use of any of claims 1-4, wherein the xenon/argon volume ratio is between 4/1 and 1/4, preferably 1/1.

6. The pharmaceutical composition for use of any of claims 1-5, wherein the volume proportion of xenon is 15%, and the volume proportion of argon is 15%.

7. The pharmaceutical composition for use of any of claims 1 to 6, wherein the gas complement comprises oxygen, nitrogen, helium, hydrogen, neon or a mixture thereof.

8. The pharmaceutical composition for use of any of claims 1 to 7, wherein the gas complement comprises oxygen in a volume proportion of between 20% and 50%, and wherein the remainder of the gas in the gas complement is preferably nitrogen, helium, hydrogen, neon or a mixture thereof.

9. The pharmaceutical composition of any of claims 1-8, wherein the pharmaceutical composition is preconditioned as a compressed gas mixture.

10. The pharmaceutical composition of any of claims 1-8, wherein the pharmaceutical composition administered to the patient is obtained by mixing argon, xenon and the gas complement in a gas mixer.

11. The pharmaceutical composition for use of any of claims 1 to 10, wherein the method for providing organ protection aims at treating and/or preventing and/or slowing degradation of organ cellular structure and/or function.

12. The pharmaceutical composition for use of any of claims 1 to 10, wherein the method is for providing neuroprotection, and aims at treating and/or preventing and/or slowing degradation of neuronal structure and/or function.

13. The pharmaceutical composition for use of claim 11 or 12, wherein the degradation of neuronal or organ cell structure and/or function is caused by a disease or condition selected from the group consisting of: hypoxic-ischemic encephalopathy, traumatic brain injury, neurocognitive disorders (in particular those induced by cardiac arrest), post-traumatic stress disorder (PTSD), neurodegenerative diseases (such as Alzheimer's Disease or Parkinson's Disease), and psychiatric disorders (such as schizophrenia, depression, bipolar disorder, addiction, anxiety disorders, attention deficit disorder and/or hyperactivity disorder).

14. The pharmaceutical composition for use of claim 11 or 12, wherein the degradation of neuronal or organ cell structure and/or function is caused by a decompression accident.
